# EUROPEAN PATENT SPECIFICATION

(11) **EP 0 656 746 B1**
(45) Date of publication and mention of the grant of the patent: **02.01.2003**
(21) Application number: 92919177.3
(22) Date of filing: 25.08.1992
(51) Int. Cl.: A01N 31/04, A61K 31/04, A61L 2/18, C11D 3/20, C11D 3/33, C11D 3/43, C11D 3/48, G02C 13/00

(54) **A soft contact lens disinfecting aqueous solution, a method for disinfecting contact lenses, and a preserved opthalmic drug solution**
Wässrige Desinfizierungslösung für weiche Kontaktlinsen, Verfahren zur Desinfizierung weicher Kontaklinsen, und preservierte Lösung opthalmischer Arzneimittel
Solution désinfectant aqueuse pour des lentilles de contact, procédé de désinfection des lentilles de contact, et solution préservée de médicaments ophtalmiques

(43) Date of publication of application: 14.06.1995
(73) Proprietor: SHERMAN, Guy, J., Mandeville, LA 70448 (US)
(72) Inventor: SHERMAN, Guy, J., Mandeville, LA 70448 (US)
(74) Representative: Pearce, Anthony Richmond
(86) International application number: PCT/US92/07205
(87) International publication number: WO 94/004028

(56) References cited:
- EP-A- 0 175 490
- EP-A- 0 358 447
- US-A- 4 421 665
- US-A- 5 141 665

## Description

### FIELD OF THE INVENTION

The present invention relates to a cold disinfecting aqueous solution for disinfecting soft contact lenses, a method of disinfecting soft contact lenses using such a solution, and to a preserved ophthalmic drug solution that avoids ocular irritation and which includes an ophthalmic drug solution such as an over-the-counter or prescription ophthalmic drug solution.

### PRIOR ART

EP-A-0358447 is mainly concerned with solutions for cleaning, conditioning and storing, or wetting, rigid gas permeable (RGP) contact lenses. However, it also discloses a daily cold disinfecting solution for soft contact lenses having the composition:-
1% by wt benzyl alcohol containing less than 100 ppm benzaldehyde; 1.0% by weight Miranol M5-1 (a surfactant); 0.12% by wt boric acid; 0.12% by wt sodium borate; 0.15% by wt sodium edetate; 0.3% by wt sodium chloride; 0.01% by wt sodium biphosphate; and 0.01% by wt sodium phosphate.

EP-A-0175490 discloses cleaning, disinfecting and wetting solutions for rigid and soft contact lenses. In particular, it discloses a sterile contact lens disinfectant composition which comprises trimethoprim, benzyl alcohol and, optimally one or more EDTA, propylene glycol and salts (bicarbonate phosphates and chlorate). The benzaldehyde content of the benzyl alcohol is unspecified.

US-A-4529535, US-A-4560491 and US-A-4626292 disclose preservative systems for incorporation into aqueous wetting and rewetting systems for contact lenses, especially silicone copolymer (gas-permeable) and soft contact lenses. The preservative systems according to US-A-4529535 and US-A-4626292 include trimethoprim, a salt of EDTA and sorbic acid or ascorbic acid as adjuvant bactericides. The preservative system according to US-A-4560491 includes trimethoprim and benzyl alcohol as an adjuvant bactericide and optionally a salt of EDTA.

### SUMMARY OF THE INVENTION

In accordance with one aspect of the present invention, there is provided a cold disinfecting aqueous solution for disinfecting soft contact lenses comprising from 0.05% to 1.0%, based on the weight of the total aqueous solution, of benzyl alcohol containing less than or equal to 100 ppm by weight of benzaldehyde; and from 0.05% to 5.0%, based on the weight of the total aqueous solution, of isopropyl alcohol.

Preferably, the benzyl alcohol is present in an amount of 0.33% by weight of the total aqueous composition. Preferably also, the isopropyl alcohol is present in an amount of about 0.1 % to about 5.0%, more preferably about 1.5%, by weight of the total composition.

Preferably, a water soluble salt of EDTA is present in an amount in the range of from about 0.025% to about 0.75% (preferably about 0.15%) by weight of the total composition.

A preferred soft contact lens disinfecting cold (ambient) temperature storing solution utilises a polyoxyalkylated nonionic detergent and has the formula (% by weight): Tween 80 (polyoxylated detergent) 0.6%, Tween 200.6%, boric acid 1.15%, sodium borate 0.4%, disodium EDTA 0.15%, isopropyl alcohol 1.5%, and high purity benzyl alcohol (less than 100 ppm benzaldehyde) 0.33%.

The foregoing solution removes micro-organism biofilm from soft contact lenses as part of the disinfecting process by storage of the soft lenses therein. While not wishing to be bound by theory, it is believed that the presence of benzyl alcohol, isopropyl alcohol, salt of EDTA and nonionic detergent results in a cold disinfecting solution that is compatible with soft contact lenses and removes biofilm. Also, it is believed that the presence of one or more detergents in the disinfecting and cleaning, storing and conditioning solutions of the present invention enhances the effectiveness of the benzyl alcohol and salt of EDTA.

In accordance with another aspect of the invention, there is provided a preserved ophthalmic drug solution comprising an aqueous ophthalmic drug solution and benzyl alcohol containing less than or equal to 100 ppm by weight of benzaldehyde, said benzyl alcohol being present in an amount of from 0.05% to 1.0% by weight of the ophthalmic drug solution for maintaining the sterility of the ophthalmic drug solution, and the ophthalmic drug solution containing less than one ppm by weight of benzaldehyde of the total weight of the ophthalmic drug solution, but not including an ophthalmic drug solution where the ophthalmic drug being preserved is solely 0.0196% by weight of vitamin A palmitate (1.7 x 10⁶I.U./gram).

Said aqueous ophthalmic solution may be an over-the-counter or a prescription ophthalmic drug solution. Examples of such drugs include dexamethasone phosphate, pilocarpine hydrochloride, and phenylephrine hydrochloride, for example. The preservative system comprises the specified benzyl alcohol which is present in an amount sufficient to preserve the sterility of the solution.

The benzyl alcohol used in accordance with the present invention should be a high purity benzyl alcohol, whether for a contact lens solution or ophthalmic drug solutions. It has been discovered that standard grades of benzyl alcohol available for pharmaceutical use contain significant amounts of benzaldehyde. Benzaldehyde is an impurity present in commercially available benzyl alcohol. While benzaldehyde does not appear to compromise the bactericidal activity of benzyl alcohol, it has been discovered that benzaldehyde, when present in contact lens solutions in sufficient concentration, acts as an eye irritant. Benzyl alcohol at high enough concentrations (about 2% by weight or more) can also act as an ocular irritant and such benzyl alcohol concentrations should be avoided.

The benzyl alcohol that is incorporated in the contact lens solutions and ophthalmic drug solutions of the present invention should be free of a benzaldehyde concentration such that when incorporated into a contact lens solution, an excessive benzaldehyde concentration would result, and is sometimes referred to herein as "high purity benzyl alcohol". As used herein "excessive benzaldehyde concentration" means that concentration of benzaldehyde which causes significant eye irritation. Usually, in accordance with the invention, the concentration of benzaldehyde in the final contact lens solution should be no more than about one ppm (by weight of the total solution) and preferably under about one ppm by weight of the total solution, and most preferably about 0.1 ppm or less by weight of the total solution.

Suitable benzyl alcohol (high purity benzyl alcohol) for use in accordance with the invention can be obtained from Akzo Chemic America of Edison, New Jersey and Stauffer Chemical Company of Westport, Connecticut, a subsidiary of Chesebrough Ponds Inc. Akzo Chemic can provide, for example, benzyl alcohol containing less than about 100 ppm benzaldehyde by weight benzyl alcohol. Methods of removing benzaldehyde from benzyl alcohol are well known to those skilled in the art. Typically, "high purity benzyl alcohol", as used in accordance with the invention will be benzyl alcohol suitable for pharmaceutical use and having a benzaldehyde concentration of less than or equal to about 100 ppm by weight of the benzyl alcohol.

An antioxidant material can be present in the composition to prevent or minimize oxidation by the benzyl alcohol. Suitable antioxidants that can be used include sodium bisulfite and various forms of Vitamin A, such as esters of Vitamin A, preferably Vitamin A palmitate, which has a biopotency of about 1.7 x 10⁶ I.U./gram.

### DESCRIPTION OF PREFERRED EMBODIMENTS

A daily cold disinfecting solution for soft contact lenses in accordance with the invention has the formula:

**TABLE 1**

| **COMPONENT** | **AMOUNT (% BY WEIGHT)** |
|---|---|
| high purity benzyl alcohol (less than 100 ppm benzaldehyde) | 0.3 |
| Monaquat P-TC detergent | 0.001-1.0 (preferably 0.005) |
| boric acid | 1.15 |
| sodium borate | 0.4 |
| trisodium edetate | 0.15 |
| Tween 20 | 0.06 |
| Tween 80 | 0.06 |
| isopropyl alcohol | 0.05 |
| USP purified water | to 100 |

The cold disinfecting solution can be used, for example, by cleaning and then storing the soft lenses in the solution during nonwearing periods, such as overnight.

A preferred cold cleaning, disinfecting, storage and rinsing solution for soft contact lenses in accordance with the invention has the formula:

**TABLE 2**

| **COMPONENT** | **AMOUNT (% BY WEIGHT)** |
|---|---|
| Tween 80 | 0.12 |
| boric acid | 1.15 |
| sodium borate | 0.4 |
| disodium EDTA or trisodium edetate | 0.15 |
| monaquat P-TC | 0.0075 |
| isopropyl alcohol | 2.5 |
| high purity benzyl alcohol (less than 100 ppm benzaldehyde) | 0.3 |
| USP purified water | to 100 |

The disinfecting solutions for soft contact lenses include from about 0.05% to 5.0% isopropyl alcohol as an adjuvant preservative and disinfectant.

## Claims

1. A cold disinfecting aqueous solution for disinfecting soft contact lenses comprising from 0.05% to 1.0%, based on the weight of the total aqueous solution, of benzyl alcohol containing less than or equal to 100 ppm by weight of benzaldehyde; and from 0.05% to 5.0%, based on the weight of the total aqueous solution, of isopropyl alcohol.

2. The solution of claim 1, further comprising from 0.025 % to 0.75% of a water soluble salt of EDTA, by weight of the total aqueous composition.

3. The solution of claim 1, wherein said benzyl alcohol is present in an amount of 0.33% by weight of the total aqueous composition.

4. The solution of claim 1, wherein said isopropyl alcohol is present in an amount of 0.1% to 5% by weight of the total aqueous composition.

5. The solution of claim 1, wherein said isopropyl alcohol is present in an amount of 1.5% by weight of the total aqueous composition.

6. The solution of claim 1, wherein said benzyl alcohol is present in an amount of 0.33% and said isopropyl alcohol is present in an amount of 1.5%, all by weight of the total aqueous composition.

7. A method of disinfecting a soft contact lens comprising contacting the lens with a composition as claimed in any preceding claim.

8. A preserved ophthalmic drug solution comprising an aqueous ophthalmic drug solution and benzyl alcohol containing less than or equal to 100 ppm by weight of benzaldehyde, said benzyl alcohol being present in an amount of from 0.05% to 1.0% by weight of the ophthalmic drug solution for maintaining the sterility of the ophthalmic drug solution, and the ophthalmic drug solution containing less than one ppm by weight of benzaldehyde of the total weight of the ophthalmic drug solution, but not including an ophthalmic drug solution where the ophthalmic drug being preserved is solely 0.0196% by weight of vitamin A palmitate (1.7 x 10⁶ I.U./gram).

## Patentansprüche

1. Kalt desinfizierende wässrige Lösung zum Desinfizieren weicher Kontaktlinsen, bezogen auf das Gesamtgewicht der wässrigen Lösung aufweisend 0,05% bis 1,0 Gewichtsprozent Benzylalkohol, der weniger oder gleich 100ppm Gewichtsteile Benzaldehyd enthält; sowie bezogen auf das Gesamtgewicht der wässrigen Lösung aufweisend 0,05% bis 5,0 Gewichtsprozent Isopropanol.

2. Lösung nach Anspruch 1, ferner bezogen auf das Gesamtgewicht der wässrigen Zusammensetzung aufweisend 0,025% bis 0,75 Gewichtsprozent eines wasserlöslichen Salzes von EDTA.

3. Lösung nach Anspruch 1, worin der Benzylalkohol in einer Menge von 0,33 Gewichtsprozent der gesamten wässrigen Zusammensetzung vorliegt.

4. Lösung nach Anspruch 1, worin das Isopropanol in einer Menge von 0,1% bis 5,0 Gewichtsprozent der gesamten wässrigen Zusammensetzung vorliegt.

5. Lösung nach Anspruch 1, worin das Isopropanol in einer Menge von 1,5 Gewichtsprozent der gesamten wässrigen Zusammensetzung vorliegt.

6. Lösung nach Anspruch 1, worin der Benzylalkohol in einer Menge von 0,33 Gewichtsprozent vorliegt und das Isopropanol in einer Menge von 1,5 Gewichtsprozent vorliegt, insgesamt bezogen auf die gesamte wässrige Zusammensetzung.

7. Verfahren zum Desinfizieren weicher Kontaktlinsen, umfassend das Kontaktieren der Linsen mit einer Zusammensetzung nach einem der vorgenannten Ansprüche.

8. Lösung eines ophthalmischen Arzneimittels, aufweisend eine wässrige Lösung eines ophthalmischen Arzneimittels und Benzylalkohol, enthaltend weniger oder gleich 100ppm Gewichtsteile Benzaldehyd, wobei der Benzylalkohol in einer Menge von 0,05% bis 1,0 Gewichtsprozent der Lösung des ophthalmischen Arzneimittels vorliegt, um die Sterilität der Lösung des ophthalmischen Arzneimittels aufrecht zu erhalten, und die Lösung des ophthalmischen Arzneimittels, enthaltend weniger als 1ppm Gewichtsteile Benzaldehyd des Gesamtgewichts der Lösung des ophthalmischen Arzneimittels, jedoch ohne Einbeziehung einer Lösung des ophthalmischen Arzneimittels, wo das ophthalmische Arzneimittel das konserviert wird ausschließlich 0,0196 Gewichtsprozent Vitamin A-Palmitat (1,7x10⁶ IU/g) ist.

## Revendications

1. Solution désinfectante aqueuse froide pour désinfecter des lentilles de contact souples, comprenant de 0,05% à 1,0%, par rapport au poids de la solution aqueuse totale, d'alcool benzylique contenant une quantité inférieure ou égale à 100ppm en poids de benzaldéhyde; et de 0,05% à 5,0%, par rapport au poids de la solution aqueuse totale, d'alcool isopropylique.

2. Solution selon la revendication 1, comprenant en outre de 0,025% à 0,75% d'un sel hydrosoluble d'EDTA, en poids de la composition aqueuse totale.

3. Solution selon la revendication 1, dans laquelle ledit alcool benzylique est présent en une quantité de 0,33% en poids de la composition aqueuse totale.

4. Solution selon la revendication 1, dans laquelle ledit alcool isopropylique est présent en une quantité de 1,5% à 5% en poids de la composition aqueuse totale.

5. Solution selon la revendication 1, dans laquelle ledit alcool isopropylique est présent en une quantité de 1,5% en poids de la composition aqueuse totale.

6. Solution selon la revendication 1, dans laquelle ledit alcool benzylique est présent en une quantité de 0,33% et ledit alcool isopropylique est présent en une quantité de 1,5%, tous en poids de la composition aqueuse totale.

7. Procédé de désinfection d'une lentille de contact souple, comprenant la mise en contact de la lentille avec une composition telle que revendiquée dans l'une quelconque des revendications précédentes.

8. Solution ophtalmique médicamenteuse préservée comprenant une solution ophtalmique médicamenteuse aqueuse et un alcool benzylique contenant une quantité inférieure ou égale à 100ppm en poids de benzaldéhyde, ledit alcool benzylique étant présent en une quantité de 0,05% à 1,0% en poids de la solution ophtalmique médicamenteuse pour maintenir la stérilité de la solution ophtalmique médicamenteuse, et la solution ophtalmique médicamenteuse contenant moins d'un ppm en poids de benzaldéhyde par rapport au poids total de la solution ophtalmique médicamenteuse, mais sans inclure une solution ophtalmique médicamenteuse dans laquelle la substance ophtalmique médicamenteuse qui est préservée est seulement à 0,0196% en poids de palmitate de vitamine A (1,7x10⁶ U.I./gramme).
